# EUROPEAN PATENT SPECIFICATION

(11) **EP 1 444 984 B1**
(45) Date of publication and mention of the grant of the patent: **10.09.2008**
(21) Application number: 04002671.8
(22) Date of filing: 06.02.2004
(51) Int. Cl.: A61K 36/61, A61K 31/728, A61K 31/10, A61P 29/00

(54) **Topical pharmaceutical compositions containing natural active constituents suitable for the prevention and treatment of mucosal inflammation processes**
Topische pharmazeutische Zusammensetzungen enthaltend aktive natürliche Bestandteile zur Verhütung und Behandlung von mucosalen Entzündungprozessen
Compositions pharmaceutiques topiques contenant des principes actifs naturels pour la prévention et le traitement des processus inflammatoires des muqueuses

(30) Priority: 06.02.2003 IT MI20030195
(43) Date of publication of application: 11.08.2004
(73) Proprietor: B.S.D. BIO SCIENCE DEVELOPMENT SNC Di OMINI C. & ZUCCARI G., 20060 Bussero (Milano) (IT)
(72) Inventor: Omini, Claudio, Franco, 20060 Bussero (MI) (IT); Zuccari, Giuseppe, 26866 Sant'Angelo Lodigiano (LO) (IT)
(74) Representative: Minoja, Fabrizio

(56) References cited:
- EP-A- 1 230 927
- EP-A- 1 236 466
- WO-A-99/34811
- US-A- 5 888 984

## Description

The present invention relates to topical pharmaceutical compositions for use in the prevention and/or treatment of inflammatory disorders of human and animal mucous membranes, in particular of the oral, anal or genital mucosae.

Numerous synthetic or semi-synthetic chemical compounds are available to treat the vast majority of inflammatory disorders. For example, non-steroidal anti-inflammatory drugs, antibiotics, local anaesthetics and disinfectants are widely used.

However, an increasing number of consumers feel the need for treatments which only use substances of natural origin.

To meet this need, numerous scientific research projects are under way which have led to the identification of numerous natural active substances suitable for the treatment or prevention of inflammatory disorders.

For example, the use of hyaluronic acid-based preparations in the treatment and prevention of periodontal disease and other disorders typical of the oral mucosa is well known in the prior art, as expressly claimed by Di Schiena in EP 0444492, and in other disorders of the epithelial surfaces, as disclosed in WO 0209637.

Numerous pharmaceutical compositions based on hyaluronic acid are currently marketed which claim an anti-inflammatory, regenerating and balancing effect on the extracellular water.

Similarly, methyl-sulphonyl-methane (MSM) has been presented as an agent which, in combination with an ordinary toothpaste, reduces gum irritation caused by the presence of plaque.

The active constituents extracted from *Melaleuca alternifolia* (the tea tree) are classically indicated and used in the prevention and treatment of numerous disorders of the oral cavity, especially ulcerative gingivitis and periodontal infections (WO 9609834).

In general, although most natural active substances are currently well identified and purified, the exact mechanism whereby they perform their pharmacological action is not a single but usually a multifactorial mechanism, i.e. the substance interacts with a number of sites in the body to produce the therapeutic action.

For example, MSM performs multiple pharmacological activities, as described by RJ Herschler in USP no. 4973605, which are able to control problems caused by gastric hyperacidity, constipation, hypersensitivity, lung disorders, parasitic infections, arthritis, hypertension, etc..

Similarly, the active constituents of *Melaleuca alternifolia* are indicated as disorders including bacterial and fungal infections, gingivitis and other oral diseases, acne, psoriasis and other skin diseases, etc..

It is therefore known that hyaluronic acid possesses a marked anti-inflammatory and gum healing activity, MSM has an analgesic effect on gingival hypersensitivity, and that the active constituents of *Melaleuca alternifolia* possess a proven antibacterial activity which is useful in the prevention and treatment of oral infections and for oral hygiene in general.

It has now surprisingly been found that when hyaluronic acid, methyl-sulphonyl-methane and tea tree extracts are combined together, they interact synergistically, thus showing a therapeutic effect much greater than that would be achieved by the use of the single components. They can therefore be advantageously used for the preparation of topical medicaments for the prevention of treatment of inflammatory diseases.

Accordingly, the present invention relates to topical pharmaceutical compositions comprising:
- hyaluronic acid or a pharmaceutically acceptable salt thereof, preferably the sodium salt;
- methyl-sulphonyl-methane and;
- *Melaleuca alternifolia* extracts
in combination with pharmaceutically acceptable excipients and/or carriers.

Hyaluronic acid can be present in amounts of between 0.01 and 2% by weight, preferably between 0.1 and 0.3%, and even more preferably 0.2%.
As herein used, hyaluronic acid means hyaluronic acid with a molecular weight ranging from 5.000 to 15.000.000 Da. In fact, although hyaluronic acid is available in various molecular weights, for the purposes of the present invention no significant differences have been observed between low molecular weight (5.000 - 300.000 Da) and high molecular weight (> 800.000 Da) hyaluronic acid. However, hyaluronic acid sodium salt obtained by biotechnology with a molecular weight of around 1.200.000 Da is particularly preferred.

MSM can be present in amounts of between 0.1 and 10% by weight, preferably between 0.5 and 5%, and even more preferably between 1 and 3%.

The *Melaleuca alternifolia* extract can be present in amounts of between 0.1 and 5% by weight. Essential oil of *Melaleuca alternifolia* (Australian tea tree oil) is particularly preferred. Of the various essential tea tree oils, the distillate rich in terpenen-4-ol is particularly preferred. Thus, according to a preferred embodiment of the invention, the compositions also comprise terpenen-4-ol in amounts of between 0.03 and 0.3 by weight, and preferably between 0.05 and 0.2.

The compositions can also contain lysozyme hydrochloride in amounts of between 0.1 and 3% by weight, and preferably between 0.3 and 1%. This composition is particularly useful in the case of concomitant labial herpes infections.

The topical compositions of the invention may be in the form of gels, creams and pastes and can contain various excipients, including viscosifying agents, surfactants, bioadhesives, co-solubilising agents and stabilisers, as well as preservatives, sweeteners, flavourings and fragrances. If required, the compositions can be wholly liquid, such as ordinary mouthwashes, vaginal lavage or anal lavage products.

Examples of suitable excipients include cellulose derivatives such as methyl- and propylcellulose, acrylate or methacrylate polymers or copolymers, glycols such as propyl and ethyl glycol, castor oil derivatives such as polyethoxylated hydrogenated castor oil, EDTA, sodium benzoate, methyl and propyl parabens, sorbic acids and the sodium or potassium salts thereof, aspartame, acesulfame, saccharine, cyclamates, neohesperidine and xylitol.

Particularly preferred compositions are gels with an aqueous base containing cellulose derivatives together with a bioadhesive agent, such as poloxamers, carbopol, polymethyl vinyl ethers and the salts and hydrocolloids thereof, such as galactomannans, pectins, alginates, carrageenans, xanthans and gelatins.

The compositions of the invention are suitable for topical use, in particular on the oral mucosae, including cosmetic hygiene of the teeth and of the mouth in general, due to their ability of acting on a number of etiopathogenetic factors involved in disorders of the mouth, lips and tongue, including tooth and periodontal disorders and dental disorders in general. Dental disorders cover a wide range of conditions, including common disorders of the lips, mouth and tongue such as stomatitis, oral herpes, angioedema of the lips, aphtha, etc., tooth disorders such as dental caries and its complications, and periodontal diseases such as gingivitis and periodontitis, including those of traumatic origin such as tooth extractions, abrasions, tooth cleaning, surgery in general, etc. (Merck Manual of Diagnosis and Treatment, 2nd Italian edition).

As already mentioned hereinbefore, the association of hyaluronic acid, MSM and tea tree extract show a synergistic interaction. Although the possibility of obtaining enhanced effects by combining two or more active constituents with different action mechanisms is known in classic pharmacology, such enhancement is not found in the case of identical action mechanisms; in particular, partial agonism is observed when two active constituents compete for the same binding site. In the case of natural active substances which, as mentioned, have multiple and potentially coinciding action mechanisms, as in this case, elision of the therapeutic actions is an important factor. The positive interaction between hyaluronic acid and MSM observed in this invention is even more surprising, as it is known to those skilled in the art that MSM is used to reduce the dehydrating action of hyaluronic acid in the connective tissue, as cited on page 7, paragraph a of USP 4477469 (MSM administered to connective tissue). Connective tissue is one of the main anatomical constituents of gingival tissue, and the positive synergistic action between hyaluronic acid and MSM constitutes a significant breakthrough in the prevention and treatment of periodontal diseases, and especially in healing processes after tooth extraction.

The compositions of the invention are also useful for topical use in all conditions involving inflammatory processes of mucosae which can be reached by means of external pharmacological treatment, such as the anal and genital mucosae. The compositions are particularly useful for the treatment or prevention of inflammatory gynaecological and/or proctological disorders and in all conditions involving vaginal dryness or vaginal or anal irritation and/or itching of any origin.

The doses of the compositions of the invention depend on the severity of the disorder and will be determined by those skilled in the art of medicine; for example, for the oral use, spreading the gel on the gums three or four times a day, usually after meals, for 1-2 weeks, leads to the disappearance of the symptoms.

In view of the low toxicity of the active constituents, longer administration periods are not contraindicated.

For the sole purpose of illustrating the teachings of this invention more clearly, we set out below some explanatory examples.

### Example 1 - Qualitative and quantitative composition per 100 g of formulation

Hyaluronic acid (sodium salt) 0.2 g; MSM 1.5 g; tea tree essential oil (titrated in terpinen-4-ol, Melaleucol) 0.1 g; hydroxyethylcellulose 1.9 g; copolymer of methyl vinyl ether and maleic anhydride Ca and Na salt (Gantrez MS 955) 0.8 g; sorbitol 4 g; glycerin 2 g; methylparaben 0.15 g; propylparaben 0.03 g; benzyl alcohol 0.5 g; EDTA 0.01 g; mint/liquorice flavouring 0.5 g; acesulfame 0.05 g; purified water q.s. for 100 g of formulate.

Two adults suffering from periodontitis with visible gingival inflammation, and in one case with tooth loosening and loss of a molar, who were treated for 7 days, 3 times a day after meals, with the formulate reported in Example 1, showed complete remission of the gingival inflammation and, in the case of loss of the tooth, complete healing of the gum.

### Example 2 - Qualitative and quantitative composition per 100 g of formulation

Hyaluronic acid (sodium salt) 0.2 g; MSM 2 g; tea tree essential oil (titrated in terpinen-4-ol, Melaleucol) 0.1 g; lysozyme hydrochloride 0.5 g; carbopol 1.3 g; propylene glycol 4 g; benzyl alcohol 1.4 g; EDTA sodium salt 0.05 g; isopropyl alcohol 2 g; NaOH 0.52 g; methylparaben 0.1 g; propylparaben 0.05 g; mint flavouring 0.5 g; acesulfame 0.05 g; purified water q.s. for 100 g.

In a patient suffering from labial herpes at an early stage, complete remission of the symptoms was observed after 3 days' administration of the formulation described in Example 2.

Those skilled in the art are aware that the etiopathogenetic factors that trigger inflammatory processes of the oral mucosae are basically similar to those affecting other anatomical structures, especially the vulvovaginal and/or anal mucosae.

We wished to establish whether this invention could also be useful in treating inflammatory gynaecological forms.

Vulvovaginitis in particular is characterised by inflammatory processes of infectious and non-infectious origin, with foul-smelling vaginal discharges, itching, irritation and local pain (Merck Manual of Diagnosis and Treatment, 2nd Italian edition pp. 1873-7).

Vulvar irritation may be caused by common factors such as the use of tight underwear, which prevents the skin from breathing and fails to absorb vaginal fluids, poor hygiene, and the use of deodorant sprays or unsuitable and irritating detergents. These cases, although very unpleasant for the woman, do not usually need pharmacological treatment; simple palliative treatment with soothing products is sufficient.

In a menopausal woman who presented vulvar itching and dryness, administration of the formulation reported in Example 1 for 14 days, twice a day (morning and night), led to complete remission of vulvar itching, with a subjective sensation of absence of vaginal dryness throughout the duration of the treatment.

## Claims

1. Topical pharmaceutical compositions for use in the prevention and/or treatment of inflammatory disorders of human and animal mucous membranes, comprising:
- hyaluronic acid or a pharmaceutically acceptable salt thereof;
- methyl-sulphonyl-methane and;
- *Melaleuca alternifolia* extracts
in combination with pharmaceutically acceptable excipients and/or carriers.

2. Compositions as claimed in claim 1, wherein hyaluronic acid has a molecular weight of between 5.000 and 15.000.000 Da.

3. Compositions as claimed in claims 1 or 2, wherein hyaluronic acid is in the form of the sodium salt.

4. Compositions as claimed in any one of claims 1 to 3, wherein hyaluronic acid is present in amounts of between 0.1% and 0.3% by weight.

5. Compositions as claimed in any one of claims 1 to 4, wherein methyl-sulphonyl-methane is present in amounts of between 0.1 % and 10% by weight.

6. Compositions as claimed in any one of claims 1 to 5, wherein *Melaleuca alternifolia* extract is present in amounts of between 0.1% and 5%.

7. Compositions as claimed in any one of claims 1 to 6 wherein the *Melaleuca alternifolia* extract is *Melaleuca alternifolia* essential oil.

8. Compositions as claimed in any one of claims 1 to 7 further comprising terpen-4-ol in amounts of between 0.03% and 0.3% by weight.

9. Compositions as claimed in any one of claims 1 to 8, further comprising lysozyme hydrochloride in amounts of between 0.1% and 3% by weight.

10. Compositions as claimed in any one of claims 1 to 9, in the form of aqueous or hydroalcoholic gels, pastes, and lipid-based or lipid creams.

11. Compositions as claimed in claims 9 or 10, for use in the prevention and/or treatment of labial herpes.

12. Use of an association of hyaluronic acid or a pharmaceutically acceptable salt thereof, methyl-sulphonyl-methane and *Melaleuca alternifolia* extracts for the preparation of a topical medicament for the prevention or treatment of inflammatory disorders.

13. Use according to claim 11 wherein the inflammatory disorder is an inflammatory disorder of the oral cavity or of the vaginal or anal mucosa.

## Patentansprüche

1. Topische pharmazeutische Zusammensetzungen für die Verwendung in der Prävention und/oder Behandlung von entzündlichen Krankheiten der mukösen Membranen von Menschen und Tieren, umfassend:
- Hyaluronsäure oder ein pharmazeutisch annehmbares Salz davon;
- Methyl-Sulphonyl-Methan und;
- Extrakte von *Melaleuca alternifolia*
in der Kombination mit pharmazeutisch annehmbaren Exzipienten und/oder Trägern.

2. Zusammensetzungen wie in Anspruch 1 beansprucht, worin die Hyaluronsäure ein Molekulargewicht zwischen 5000 und 15000000 Da aufweist.

3. Zusammensetzungen wie in den Ansprüchen 1 oder 2 beansprucht, worin die Hyaluronsäure in der Form des Natriumsalzes vorliegt.

4. Zusammensetzungen wie in einem der Ansprüche 1 bis 3 beansprucht, worin die Hyaluronsäure in Mengen zwischen 0,1 und 0,3 Gew.-% zugegen ist.

5. Zusammensetzungen wie in einem der Ansprüche 1 bis 4 beansprucht, worin Methyl-Sulphonyl-Methan in Mengen zwischen 0,1 und 10 Gew.-% zugegen ist.

6. Zusammensetzungen wie in einem der Ansprüche 1 bis 5 beansprucht, worin die Extrakte von *Melaleuca alternifolia* in Mengen zwischen 0,1 und 5 % zugegen ist.

7. Zusammensetzungen wie in einem der Ansprüche 1 bis 6 beansprucht, worin der Extrakt von *Melaleuca alternifolia* gleich dem essentiellen Öl *Melaleuca alternifolia* ist.

8. Zusammensetzungen wie in einem der Ansprüche 1 bis 7 beansprucht, welche darüber hinaus Terpen-4-ol in Mengen zwischen 0,03 und 0,3 Gew.-% umfassen.

9. Zusammensetzungen wie in einem der Ansprüche 1 bis 8 beansprucht, welche darüber hinaus Lysozymhydrochlorid in Mengen zwischen 0,1 und 3 Gew.-% umfassen.

10. Zusammensetzungen wie in einem der Ansprüche 1 bis 9 beansprucht in der Form von wässrigen oder hydroalkoholischen Gelen, Pasten und lipidbasierenden oder lipiden Cremes.

11. Zusammensetzungen wie in den Ansprüchen 9 oder 10 beansprucht, für die Verwendung in dem Schutz und/oder Behandlung von Lippenherpes.

12. Verwendung einer Kombination von Hyaluronsäure oder einem pharmazeutisch annehmbaren Salzes davon, Methyl-Sulphonyl-Methan und Extrakten von *Melaleuca alternifolia* für die Herstellung eines topischen Arzneimittels für die Schutz oder Behandlung von entzündungsbedingten Krankheiten.

13. Verwendung gemäß Anspruch 11, worin die entzündungsbasierende Krankheit eine Entzündungserkrankung der oralen Kavität oder der vaginalen oder analen Schleimhaut ist.

## Revendications

1. Compositions pharmaceutiques topiques destinées à une utilisation dans la prévention et/ou le traitement de troubles inflammatoires des membranes muqueuses humaines et animales, comprenant :
- de l'acide hyaluronique ou un sel de celui-ci acceptable sur le plan pharmaceutique;
- du méthyl-sulfonyl-méthane et
- des extraits de *Melaleuca alternifolia*
en combinaison avec des excipients et/ou des véhicules acceptables sur le plan pharmaceutique.

2. Compositions selon la revendication 1, dans lesquelles l'acide hyaluronique présente une masse moléculaire comprise entre 5 000 et 15 000 000 Da.

3. Compositions selon les revendications 1 ou 2, dans lesquelles l'acide hyaluronique est sous forme du sel sodique.

4. Compositions selon l'une quelconque des revendications 1 à 3, dans lesquelles l'acide hyaluronique est présent en des quantités comprises entre 0,1% et 0,3% en poids.

5. Compositions selon l'une quelconque des revendications 1 à 4, dans lesquelles le méthyl-sulfonyl-méthane est présent en des quantités comprises entre 0,1% et 10% en poids.

6. Compositions selon l'une quelconque des revendications 1 à 5, dans lesquelles l'extrait de *Melaleuca alternifolia* est présent en des quantités comprises entre 0,1 % est 5%.

7. Compositions selon l'une quelconque des revendications 1 à 6, dans lesquelles l'extrait de *Melaleuca alternifolia* est une huile essentielle de *Melaleuca alternifolia.*

8. Compositions selon l'une quelconque des revendications 1 à 7, comprenant en outre du terpén-4-ol en des quantités comprises entre 0,03% et 0,3% en poids.

9. Compositions selon l'une quelconque des revendications 1 à 8, comprenant en outre du chlorhydrate de lysozyme en des quantités comprises entre 0,1% et 3% en poids.

10. Compositions selon l'une quelconque des revendications 1 à 9, sous forme de gels aqueux ou hydroalcooliques, de pâtes, et de crèmes à base de lipides ou lipidiques.

11. Compositions selon les revendications 9 ou 10, destinées à une utilisation dans la prévention et/ou le traitement de l'herpès labial.

12. Utilisation d'une association d'acide hyaluronique ou d'un sel de celui-ci acceptable sur le plan pharmaceutique, de méthyl-sulfonyl-méthane et d'extraits de *Melaleuca alternifolia* pour la préparation d'un médicament topique destiné à la prévention ou au traitement de troubles inflammatoires.

13. Utilisation selon la revendication 11, dans laquelle le trouble inflammatoire est un trouble inflammatoire de la cavité orale ou de la muqueuse vaginale ou anale.
